# EUROPEAN PATENT APPLICATION

(11) **EP 4 742 157 A1**
(43) Date of publication of application: **13.05.2026**
(21) Application number: 24850955.6
(22) Date of filing: 05.08.2024
(51) Int. Cl.: G06T 7/70

(54) **SURGICAL CART POSITIONING METHOD, APPARATUS AND SYSTEM, AND STORAGE MEDIUM**

(30) Priority: 10.08.2023 CN 202311006826
(71) Applicant: Ronovo (Shanghai) Medical Science and Technology Ltd., Shanghai 201102 (CN)
(72) Inventor: CHEN, Zike, Shanghai 201102 (CN); MAO, Ying, Shanghai 201102 (CN)
(74) Representative: Murgitroyd & Company
(86) International application number: PCT/CN2024/109712
(87) International publication number: WO 2025/031310

(57) **Abstract**

The present application discloses a surgical cart positioning method, apparatus and system, and a storage medium. The method comprises: acquiring a first field-of-view image acquired by an image acquisition apparatus and in which a first surgical cart corresponds to a registration apparatus, and on the basis of the first field-of-view image, determining a first relative pose of the first surgical cart relative to the registration apparatus (S210); acquiring a second field-of-view image acquired by the image acquisition apparatus and in which a second surgical cart corresponds to the registration apparatus, and on the basis of the second field-of-view image, determining a second relative pose of the registration apparatus relative to the second surgical cart (S220); and on the basis of the first relative pose and the second relative pose, determining a relative positioning pose of the first surgical cart relative to the second surgical cart (S230).

## Description

The present application claims priority to Chinese patent application No. 202311006826.4, filed with China National Intellectual Property Administration on August 10, 2023, which is hereby incorporated by reference in its entirety.

### TECHNICAL FIELD

The present application relates to the technical field of medical apparatus, exemplarily, to a positioning method, a positioning apparatus and a positioning system for surgical cart, and a storage medium.

### BACKGROUND

With improvements in medical standards, minimally invasive surgery has been widely applied gradually. To ensure the feasibility and safety of the minimally invasive surgery, surgical robot systems are increasingly used in the minimally invasive surgery. In scenarios of minimally invasive surgery, in a split multi-column surgical robot system, multiple surgical carts need to be arranged around an operating table. Therefore, it is necessary to accurately position the relative pose of each two surgical carts.

Positioning devices have high requirements for environment and installation. Positioning methods in related art typically involve arranging signal transmitters and signal receivers on the surgical carts respectively. However, multiple sensor devices are prone to generating interfering information, leading to inaccurate positioning of the surgical carts. Moreover, the positioning methods in the related art are not able to adapt to scenarios of various operating rooms.

### SUMMARY

Embodiments of the present application provide a positioning method, a positioning apparatus and a positioning system for surgical cart, and a storage medium, to avoid data interference between multiple sensor devices in positioning methods in the related art and improve the positioning accuracy of surgical carts.

A positioning method for surgical cart is provided in an embodiment of the present application, and the method includes:
obtaining a first field-of-view (FOV) image correlated with a first surgical cart and a registration apparatus and collected by an image collection apparatus, and determining a first relative pose of the first surgical cart relative to the registration apparatus based on the first FOV image;
obtaining a second FOV image correlated with a second surgical cart and the registration apparatus and collected by the image collection apparatus, and determining a second relative pose of the registration apparatus relative to the second surgical cart based on the second FOV image; and
determining a relative positioning pose of the first surgical cart relative to the second surgical cart based on the first relative pose and the second relative pose.

A positioning apparatus for surgical cart is provided in another embodiment of the present application, and the apparatus includes:
a first relative pose determining module, configured to obtain a first field-of-view (FOV) image correlated with a first surgical cart and a registration apparatus and collected by an image collection apparatus, and determine a first relative pose of the first surgical cart relative to the registration apparatus based on the first FOV image;
a second relative pose determining module, configured to obtain a second FOV image correlated with a second surgical cart and the registration apparatus and collected by the image collection apparatus, and determine a second relative pose of the registration apparatus relative to the second surgical cart based on the second FOV image; and
a relative positioning pose determining module, configured to determine a relative positioning pose of the first surgical cart relative to the second surgical cart based on the first relative pose and the second relative pose.

A positioning system for surgical cart is provided in still another embodiment of the present application, and the positioning system for surgical cart includes a first surgical cart, a second surgical cart, an image collection apparatus, a registration apparatus, and a control device. The image collection apparatus is configured to a first FOV image correlated with the first surgical cart and the registration apparatus, and collect a second FOV image correlated with the second surgical cart and the registration apparatus. The control device includes at least one processor, and a memory communicating with the at least one processor. A computer program executable by the at least one processor is stored in the memory. The computer program, when executed by the at least one processor, causes the at least one processor to execute the positioning method for surgical cart according to any embodiment of the present application.

A computer-readable storage medium is provided in an embodiment of the present application. Computer instructions are stored on the computer-readable storage medium, and the computer instructions, when executed by a processor, are configured to execute the positioning method for surgical cart according to any embodiment of the present application.

### BRIEF DESCRIPTION OF THE DRAWINGS

FIG. 1 is a schematic view showing a structure of a positioning system for surgical cart according to an embodiment of the present application.
FIG. 2 is a schematic view showing a structure of another positioning system for surgical cart according to an embodiment of the present application.
FIG. 3 is a schematic view showing a structure of another positioning system for surgical cart according to an embodiment of the present application.
FIG. 4 is a schematic view showing a structure of another positioning system for surgical cart according to an embodiment of the present application.
FIG. 5 is a schematic view showing a structure of a control device according to an embodiment of the present application.
FIG. 6 is a flowchart of a positioning method for surgical cart according to an embodiment of the present application.
FIG. 7 is a flowchart of another positioning method for surgical cart according to an embodiment of the present application.
FIG. 8 is a flowchart of another positioning method for surgical cart according to an embodiment of the present application.
FIG. 9 is a schematic view showing a structure of a positioning apparatus for surgical carts according to an embodiment of the present application.

### DETAILED DESCRIPTION OF THE EMBODIMENTS

It should be noted that the terms "first", "second", etc. in the specification, claims and the above-mentioned drawings of the present application are used to distinguish similar objects, and are not necessarily used to describe a specific order or sequence. It should be understood that the data used in this way may be interchangeable where appropriate, so that the embodiments of the present invention described herein can be implemented in an order other than those illustrated or described herein. In addition, the terms "include" and "have" and any variations thereof are intended to cover non-exclusive inclusions, exemplarily, a process, method, system, product or device including a series of steps or units is not necessarily limited to those steps or units clearly listed, but may include other steps or units that are not clearly listed or that are inherent to these processes, methods, products or devices.

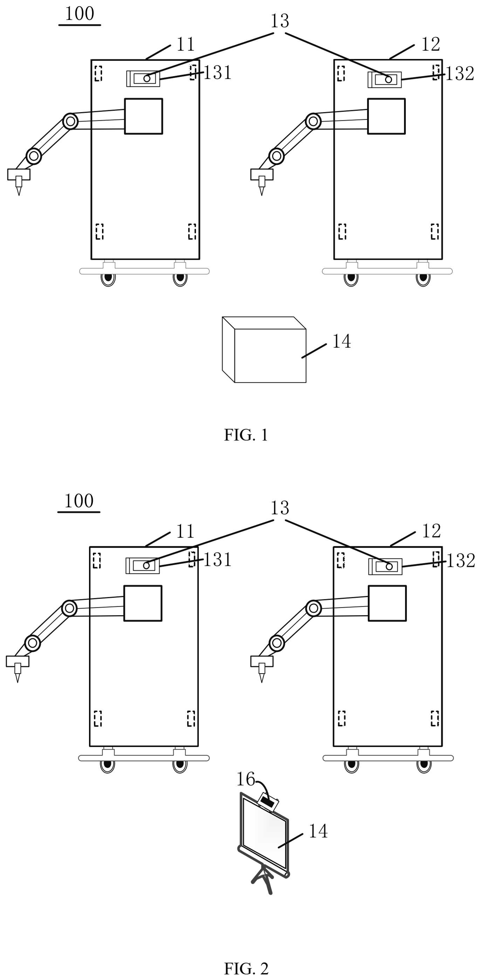

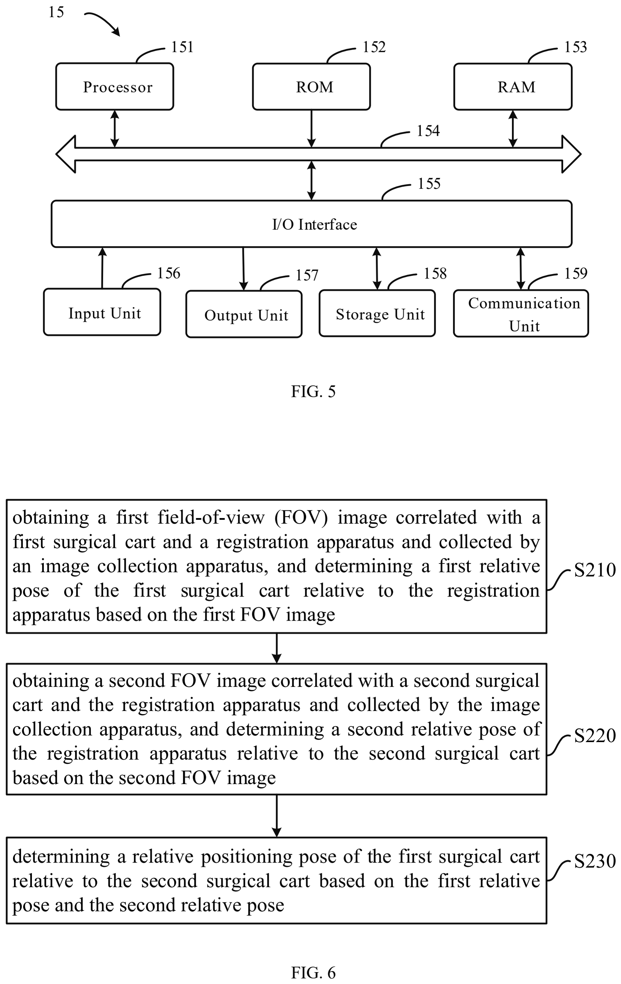

FIG. 1 is a schematic view showing a structure of a positioning system for surgical cart according to an embodiment of the present application. As shown in FIG. 1 and FIG. 5, the positioning system 100 for surgical cart includes a first surgical cart 11, a second surgical cart 12, an image collection apparatus 13, a registration apparatus 14, and a control device 15.

Exemplarily, the image collection apparatus 13 and the control device 15 are in communication. The control device 15 can be provided with a positioning apparatus for surgical cart proposed in the embodiments of the present application below to provide a service for a positioning method for surgical cart proposed in the embodiments of the present application below.

In the embodiment shown in FIG. 1, the registration apparatus 14 includes a registration device. The image collection apparatus 13 includes an image collection device 131 and another image collection device 132. The image collection device 131 is arranged on the first surgical cart 11, and the image collection device 132 is arranged on the second surgical cart 12. In this embodiment, the registration apparatus 14 refers to the registration device.

Exemplarily, a first field-of-view (FOV) image collected by the image collection device 131 and a second field-of-view (FOV) image collected by the image collection device 132 each contain the registration apparatus 14.

Based on the embodiment shown in FIG. 1, exemplarily, at least one identification pattern is provided in the registration apparatus 14, or the registration apparatus 14 has a display function for at least one identification pattern.

Exemplarily, image content of the identification pattern may be a grid, a checkerboard, a barcode, a QR code, etc. When the image content is the barcode or the QR code, the control device 15 can also be configured to perform a decoding operation on the identification pattern in the FOV image to obtain attribute data of the registration apparatus 14, such as the real-time registration pose of the registration apparatus 14.

The identification pattern is provided in the embodiment of the present application, and compared with registration based on the registration apparatus 14 in the FOV image, the registration based on the identification pattern in the FOV image can improve the accuracy of the subsequent relative positioning pose between the first surgical cart 11 and the second surgical cart 12.

Exemplarily, when the registration apparatus 14 has the display function for the at least one identification pattern, the registration apparatus 14 can be a mobile smartphone or a display screen.

In an embodiment, when the number of identification patterns is one, the first FOV image collected by the image collection device 131 and the second FOV image collected by the image collection device 132 each contain the identification pattern.

In another embodiment, when the number of the identification patterns is at least two, setting orientations (or display orientations) of the multiple identification patterns on the registration apparatus 14 are different. The identification pattern contained in the first FOV image collected by the image collection device 131 and the identification pattern contained in the second FOV image collected by the image collection device 132 may be the same or different.

When the identification patterns contained in the first FOV image and in the second FOV image respectively are different, exemplarily, an identification pattern A is disposed (or displayed) on the left-side surface of the registration apparatus 14, and an identification pattern B is disposed (or displayed) on the right-side surface of the registration apparatus 14. Alternative, the identification pattern A is disposed (or displayed) on the front-side surface of the registration apparatus 14, and the identification pattern B is disposed (or displayed) on the right-side surface of the registration apparatus 14. The specific setting orientations (or display orientations) of the multiple identification patterns can be adjusted according to an orientational range of the first surgical cart 11 and the second surgical cart 12 relative to the registration apparatus 14.

By providing (or displaying) the multiple identification patterns, when the first surgical cart 11 and the second surgical cart 12 are positioned in different orientations relative to the registration apparatus 14, it can be ensured that both the image collection device 131 and the image collection device 132 can collect FOV images containing the identification pattern, broadening the applicable scenarios of the embodiments of the present application.

FIG. 2 is a schematic view showing a structure of another positioning system for surgical cart according to an embodiment of the present application. FIG. 2 shows an example that the registration apparatus 14 is a display screen.

Based on the above embodiments, exemplarily, when the number of the identification patterns is one, a pose collecting module 16 is arranged on the registration apparatus 14. The pose collecting module 16 is configured to collect a real-time registration pose of the registration apparatus 14. Exemplarily, the pose collecting module 16 can be an Inertial Measurement Unit (IMU).

When the first surgical cart 11 and the second surgical cart 12 are positioned in different orientations relative to the registration apparatus 14, by changing the pose of the registration apparatus 14, while ensuring that both the image collection device 131 and the image collection device 132 can collect FOV images containing the identification pattern, broadening the applicable scenarios of the embodiments of the present application, the relative positioning pose of the first surgical cart relative to the second surgical cart can be determined based on the real-time registration pose collected by the pose collecting module 142, the first FOV image, and the second FOV image.

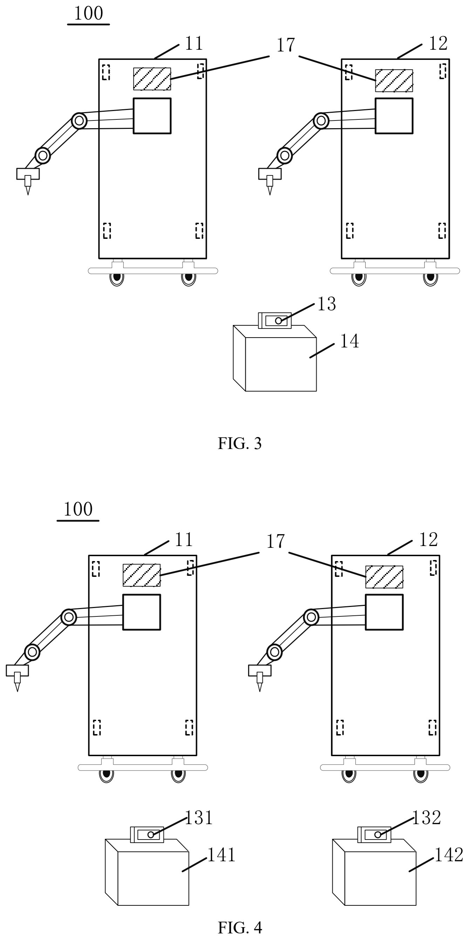

FIG. 3 is a schematic view showing a structure of another positioning system for surgical cart according to an embodiment of the present application. In the embodiment shown in FIG. 3, the registration apparatus 14 includes a registration device. The number of image collection devices in the image collection apparatus 13 is one. The image collection apparatus 13 is arranged on the registration apparatus 14. An identification pattern 17 is disposed on each of the first surgical cart 11 and the second surgical cart 12. The first FOV image is an FOV image containing the identification pattern 17 on the first surgical cart 11. The second FOV image is an FOV image containing the identification pattern 17 on the second surgical cart 12.

FIG. 4 is a schematic view showing a structure of another positioning system for surgical cart according to an embodiment of the present application. In the embodiment shown in FIG. 4, the registration apparatus 14 includes a registration device 141 and a registration device 142 which have different registration poses. The image collection apparatus 13 includes an image collection device 131 and an image collection device 132. The image collection device 131 is arranged on the registration device 141, and the image collection device 132 is arranged on the registration device 142. An identification pattern 17 is disposed on each of the first surgical cart 11 and the second surgical cart 12.

Exemplarily, the registration apparatus 14 includes the registration device 141 and registration device 142 which have different registration poses. The image collection apparatus 13 includes the image collection device 131 and image collection device 132. An identification pattern 17 can be disposed (or displayed) on each of the registration device 141 and the registration device 142. The image collection device 131 is arranged on the first surgical cart 11. The image collection device 132 is arranged on the second surgical cart 12.

It should be noted that the above embodiments are described by taking two surgical carts as examples. When the number of surgical carts is more than two, the number of the image collection devices in the image collection apparatus 13 is set corresponding to the number of the surgical carts, and/or the number of the identification patterns is set corresponding to the number of the surgical carts, and/or the number of the registration devices in the registration apparatus 14 is set corresponding to the number of the surgical carts.

FIG. 5 is a schematic view showing a structure of a control device according to an embodiment of the present application. The control device 15 is intended to represent various forms of digital computers, such as laptop computers, desktop computers, workstations, personal digital assistants, servers, blade servers, mainframe computers, and other suitable computers. An electronic device may also represent various forms of mobile devices, such as personal digital assistants, cellular phones, smartphones, wearable devices (such as helmets, glasses, watches), and other similar computing devices. The components, and connections and relationships thereof, and functions thereof shown herein are merely illustrative and are not intended to limit the implementation of the present application described and/or claimed herein.

As shown in FIG. 5, the control device 15 includes at least one processor 151 and a memory communicating with the at least one processor 151, such as a Read-Only Memory (ROM) 152, a Random Access Memory (RAM) 153. The memory stores a computer program executable by the at least one processor 151. The computer program, when executed by the at least one processor 151, causes the at least one processor 151 to execute the positioning method for surgical cart provided in the embodiments above. The processor 151 can execute various appropriate actions and processes according to the computer program stored in the ROM 152 or the computer program loaded into the RAM 153 from a storage unit 158. Various programs and data required for operations of the control device 15 can be stored in the RAM 153. The processor 151, the ROM 152, and the RAM 153 are connected to each other through a bus 154. An Input/Output (I/O) interface 155 is also connected to the bus 154.

A plurality of components in the control device 15 are connected to the I/O interface 155, and include: an input unit 156, such as a keyboard or a mouse; an output unit 157, such as one of various types of displays, speakers; a storage unit 158, such as a magnetic disk or an optical disk; and a communication unit 159, such as a network card, a modem, or a wireless communication transceiver. The communication unit 159 allows the control device 15 to exchange information/data with other devices through computer networks such as the Internet and/or various telecommunication networks.

The processor 151 may be one of various general-purpose and/or special-purpose processing components having processing and computing capabilities. Some examples of the processor 151 include, but are not limited to, a Central Processing Unit (CPU), a Graphics Processing Unit (GPU), various specialized Artificial Intelligence (AI) computing chips, various processors running machine learning model algorithms, a Digital Signal Processor (DSP), and any suitable processor, controller, microcontroller, etc. The processor 151 executes the various methods and processes described above, such as the positioning method for surgical cart provided in the embodiments above.

In some embodiments, the positioning method for surgical cart provided in the embodiments above can be implemented as a computer program, which is tangibly contained in a computer-readable storage medium, such as the storage unit 158. In some embodiments, part or all of the computer program can be loaded and/or installed onto the control device 15 through the ROM 152 and/or the communication unit 159. When the computer program is loaded into the RAM 153 and executed by the processor 151, one or more steps of the positioning method for surgical cart described above can be executed. Exemplarily, in other embodiments, the processor 151 may be configured to execute the positioning method for surgical cart in any other suitable manners (e.g., by means of firmware).

The various implementations of the systems and techniques described above herein can be implemented in digital electronic circuitry, integrated circuit systems, field programmable gate arrays (FPGAs), application specific integrated circuits (ASICs), application specific standard products (ASSPs), systems on a chip system (SOCs), complex programmable logic devices (CPLDs), computer hardware, firmware, software, and/or combinations thereof. These various implementations may include implementations in one or more computer programs executable and/or interpretations on a programmable system including at least one programmable processor, which may be a special-purpose or general-purpose programmable processor, capable of receiving data and instructions from, and transmitting data and instructions to, a storage system, at least one input device, and at least one output device.

The computer programs for implementing the positioning method for surgical cart of the present application can be written in any combination of one or more programming languages. These computer programs can be provided to a processor of a general-purpose computer, a special-purpose computer, or other programmable data processing apparatuses, such that the computer program, when executed by the processor, causes the functions/operations specified in the flowcharts and/or block diagrams to be implemented. The computer programs can be executed entirely on the machine, partly on the machine, partly on the machine as a stand-alone software package and partly on a remote machine, or entirely on the remote machine or server.

FIG. 6 is a flowchart of a positioning method for surgical cart according to an embodiment of the present application. This embodiment is applicable to situations where the relative pose relationship between multiple surgical carts needs to be positioned. This method can be executed by a positioning apparatus for surgical cart. The positioning apparatus for surgical cart can be implemented in hardware and/or software and configured in a control device. As shown in FIG. 6, the method includes the following steps.

S210, obtain a first FOV image, correlated with a first surgical cart and a registration apparatus and collected by an image collection apparatus, and determine a first relative pose of the first surgical cart relative to the registration apparatus based on the first FOV image.

In this embodiment, the registration apparatus includes a registration device.

Exemplarily, obtaining the first FOV image correlated with the first surgical cart and the registration apparatus collected by the image collection apparatus, includes obtaining the first FOV image containing the registration apparatus and collected by an image collection device arranged on the first surgical cart.

Exemplarily, obtaining the first FOV image correlated with the first surgical cart and the registration apparatus collected by the image collection apparatus, includes obtaining the first FOV image containing an identification pattern and collected by an image collection device arranged on the first surgical cart. The identification pattern is disposed on the registration apparatus.

Exemplarily, obtaining the first FOV image correlated with the first surgical cart and the registration apparatus collected by the image collection apparatus, includes obtaining the first FOV image containing an identification pattern and collected by an image collection device arranged on the registration apparatus. The identification pattern is disposed on the first surgical cart.

Exemplarily, determining the first relative pose of the first surgical cart relative to the registration apparatus based on the first FOV image includes: obtaining object pixel data of a target object in the first FOV image, and determining the first relative pose of the first surgical cart relative to the registration apparatus by using an Efficient Perspective-n-Point (EPnP) camera pose estimation algorithm, based on the object pixel data, and a camera intrinsic matrix and a distortion matrix of the image collection apparatus. The target object is the registration apparatus or the identification pattern provided on the registration apparatus.

Exemplarily, the camera intrinsic matrix and the distortion matrix are respectively the camera intrinsic matrix and the distortion matrix of the image collection device in the image collection apparatus collecting the first FOV image.

Exemplarily, determining the first relative pose of the first surgical cart relative to the registration apparatus based on the first FOV image includes: obtaining object pixel data of a target object in the first FOV image, determining a homography transformation matrix based on calibrated pixel data and the object pixel data, and determining the first relative pose of the first surgical cart relative to the registration apparatus based on the homography transformation matrix.

Exemplarily, the calibrated pixel data characterizes camera calibration data of the image collection device in the image collection apparatus that collects the first FOV image.

S220, obtain a second FOV image, correlated with a second surgical cart and the registration apparatus and collected by the image collection apparatus, and determine a second relative pose of the registration apparatus relative to the second surgical cart based on the second FOV image.

Exemplarily, obtaining the second FOV image correlated with the second surgical cart and the registration apparatus and collected by the image collection apparatus, includes obtaining the second FOV image containing the registration device and collected by an image collection device arranged on the second surgical cart.

Exemplarily, obtaining the second FOV image correlated with the second surgical cart and the registration apparatus and collected by the image collection apparatus, includes obtaining the second FOV image containing an identification pattern and collected by an image collection device arranged on the second surgical cart. The identification pattern is disposed on the registration device.

Exemplarily, obtaining the second FOV image correlated with the second surgical cart and the registration apparatus and collected by the image collection apparatus, includes obtaining the second FOV image containing an identification pattern and collected by an image collection device arranged on the registration apparatus. The identification pattern is disposed on the second surgical cart.

Exemplarily, determining the second relative pose of the registration apparatus relative to the second surgical cart based on the second FOV image includes: obtaining object pixel data of a target object in the second FOV image, and determining the second relative pose of the registration device relative to the second surgical cart by using the Efficient Perspective-n-Point (EPnP) camera pose estimation algorithm, based on the object pixel data, and a camera intrinsic matrix and a distortion matrix of the image collection device. The target object is the registration apparatus or the identification pattern provided on the registration apparatus.

Exemplarily, the camera intrinsic matrix and the distortion matrix are respectively the camera intrinsic matrix and the distortion matrix of the image collection device in the image collection apparatus collecting the second FOV image.

Exemplarily, determining the second relative pose of the registration apparatus relative to the second surgical cart based on the second FOV image includes: obtaining object pixel data of a target object in the second FOV image, determining a homography transformation matrix based on calibrated pixel data and the object pixel data, and determining the second relative pose of the registration device relative to the second surgical cart based on the homography transformation matrix.

Exemplarily, the calibrated pixel data characterizes camera calibration data corresponding to the image collection device in the image collection apparatus collecting the second FOV image.

S230, determine a relative positioning pose of the first surgical cart relative to the second surgical cart based on the first relative pose and the second relative pose.

In this embodiment, exemplarily, the relative positioning pose of the first surgical cart relative to the second surgical cart can be obtained by adding the first relative pose and the second relative pose.

In the technical solutions of the embodiments, the first FOV image correlated with the first surgical cart and the registration apparatus and collected by an image collection apparatus is obtained, and the first relative pose of the first surgical cart relative to the registration apparatus is determined based on the first FOV image, the second FOV image correlated with a second surgical cart and the registration apparatus and collected by the image collection apparatus is obtained, and the second relative pose of the registration apparatus relative to the second surgical cart is determined based on the second FOV image, and the relative positioning pose of the first surgical cart relative to the second surgical cart is determined based on the first relative pose and the second relative pose, thereby avoiding the data interference between multiple sensor devices in positioning methods of the related art, and improving the positioning accuracy of the surgical carts while reducing the positioning complexity.

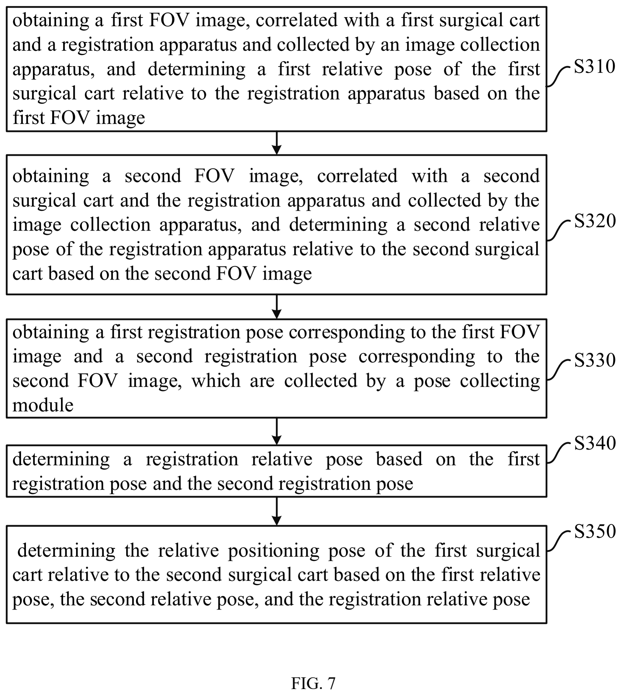

FIG. 7 is a flowchart of another positioning method for surgical cart according to an embodiment of the present application. The embodiment further limits step 230 in the above embodiments specifically. As shown in FIG. 7, the method includes the following steps.

S310, obtain a first FOV image, correlated with a first surgical cart and a registration apparatus and collected by an image collection apparatus, and determine a first relative pose of the first surgical cart relative to the registration apparatus based on the first FOV image.

S320, obtain a second FOV image, correlated with a second surgical cart and the registration apparatus and collected by the image collection apparatus, and determine a second relative pose of the registration apparatus relative to the second surgical cart based on the second FOV image.

Step S310 and step S320 in this embodiment are the same as or similar to step S210 and step S220 respectively shown in FIG. 6 in the above embodiments, which are not repeatedly described herein.

S330, obtain a first registration pose corresponding to the first FOV image and a second registration pose corresponding to the second FOV image, which are collected by a pose collecting module.

In an embodiment, a real-time registration pose of a registration device in the registration apparatus may change.

Exemplarily, the pose collecting module is configured to collect the real-time registration pose of the registration device. The first registration pose characterizes a real-time registration pose of the registration device corresponding to a collection time of the first FOV image. The second registration pose characterizes a real-time registration pose of the registration device corresponding to a collection time of the second FOV image.

S340, determine a registration relative pose based on the first registration pose and the second registration pose.

Exemplarily, determining the registration relative pose based on the first registration pose and the second registration pose includes: obtaining a change time interval corresponding to the first registration pose and the second registration pose, and a motion angular velocity and a motion acceleration; obtaining an angular displacement of the registration relative pose by performing integration on the motion angular velocity over the change time interval; obtaining a translational displacement of the registration relative pose by performing double integration on the motion acceleration over the change time interval. The registration relative pose can be characterized by the angular displacement and the translational displacement.

Exemplarily, the change time interval characterizes a time interval between the collection time of the first FOV image and the collection time of the second FOV image.

Based on the above embodiments, exemplarily, it is determined whether the first registration pose and the second registration pose are the same. If the first registration pose and the second registration pose are the same, the registration relative pose is set to null. If the first registration pose and the second registration pose are different, perform the step of determining the registration relative pose based on the first registration pose and the second registration pose.

S350, determine the relative positioning pose of the first surgical cart relative to the second surgical cart based on the first relative pose, the second relative pose, and the registration relative pose.

In an embodiment, exemplarily, the relative positioning pose of the first surgical cart relative to the second surgical cart can be obtained by adding the first relative pose, the second relative pose, and the registration relative pose.

In the technical solutions of the embodiments, the pose collecting module is arranged in the registration apparatus, the registration relative pose is determined based on the first registration pose corresponding to the first FOV image and the second registration pose corresponding to the second FOV image which are collected by the pose collecting module, and the relative positioning pose of the first surgical cart relative to the second surgical cart is determined based on the first relative pose, the second relative pose, and the registration relative pose, thereby avoiding the case where the image collection apparatus cannot collect the FOV images containing the target object due to the limitations of the field of view of the image collection apparatus, improving the flexibility of the positioning method for surgical cart, and broadening the applicable scenarios of the embodiments of the present application.

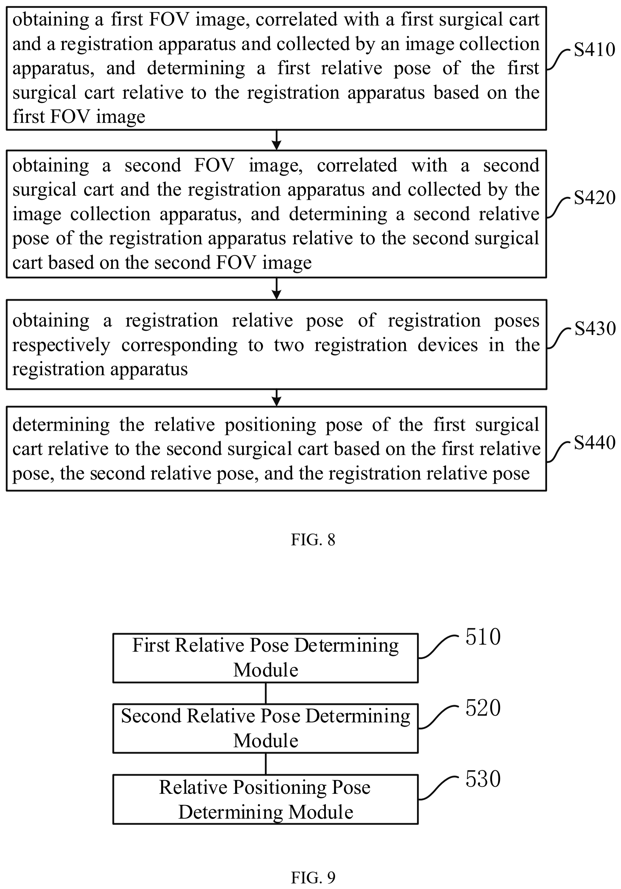

FIG. 8 is a flowchart of another positioning method for surgical cart according to an embodiment of the present application. As shown in FIG. 8, the method includes the following steps.

S410, obtain a first FOV image, correlated with a first surgical cart and a registration apparatus and collected by an image collection apparatus, and determine a first relative pose of the first surgical cart relative to the registration apparatus based on the first FOV image.

In this embodiment, the registration apparatus includes two registration devices with different registration poses.

Exemplarily, obtaining the first FOV image correlated with the first surgical cart and the registration apparatus collected by the image collection apparatus, includes obtaining the first FOV image containing the registration apparatus with a first registration pose and collected by the image collection device arranged on the first surgical cart.

Exemplarily, obtaining the first FOV image correlated with the first surgical cart and the registration apparatus collected by the image collection apparatus, includes obtaining the first FOV image containing an identification pattern and collected by the image collection device arranged on the first surgical cart. The identification pattern is disposed on the registration device with the first registration pose.

Exemplarily, obtaining the first FOV image correlated with the first surgical cart and the registration apparatus collected by the image collection apparatus, includes obtaining the first FOV image containing an identification pattern and collected by an image collection device arranged on the registration device with the first registration pose. The identification pattern is disposed on the first surgical cart.

The performing logic of "determining the first relative pose of the first surgical cart relative to the registration apparatus based on the first FOV image" in this embodiment is the same as or similar to the performing logic of "determining the first relative pose of the first surgical cart relative to the registration apparatus based on the first FOV image" in the above embodiments, and is not repeatedly described herein.

S420, obtain a second FOV image, correlated with a second surgical cart and the registration apparatus and collected by the image collection apparatus, and determine a second relative pose of the registration apparatus relative to the second surgical cart based on the second FOV image.

Exemplarily, obtaining the second FOV image correlated with the second surgical cart and the registration apparatus and collected by the image collection apparatus includes: obtaining the second FOV image containing a registration device with a second registration pose and collected by an image collection device arranged on the second surgical cart.

Exemplarily, obtaining the second FOV image correlated with the second surgical cart and the registration apparatus and collected by the image collection apparatus includes: obtaining the second FOV image containing an identification pattern and collected by an image collection device arranged on the second surgical cart. The identification pattern is disposed on the registration device with the second registration pose.

Exemplarily, obtaining the second FOV image correlated with the second surgical cart and the registration apparatus and collected by the image collection apparatus includes: obtaining the second FOV image containing an identification pattern and collected by an image collection device arranged on the registration device with the second registration pose. The identification pattern is disposed on the second surgical cart.

The performing logic of "determining the second relative pose of the second surgical cart relative to the registration apparatus based on the second FOV image" in this embodiment is the same as or similar to the performing logic of "determining the second relative pose of the second surgical cart relative to the registration apparatus based on the second FOV image" in the above embodiments, and is not repeatedly described herein.

S430, obtain a registration relative pose of registration poses respectively corresponding to two registration devices in the registration apparatus.

In an embodiment, the respective registration poses of the two registration devices remain unchanged during the positioning of the surgical carts. The registration relative pose may be preset before performing the positioning method for surgical cart.

S440, determine the relative positioning pose of the first surgical cart relative to the second surgical cart based on the first relative pose, the second relative pose, and the registration relative pose.

In an embodiment, exemplarily, the relative positioning pose of the first surgical cart relative to the second surgical cart can be obtained by adding the first relative pose, the second relative pose, and the registration relative pose.

In the technical solutions of the embodiments, two registration devices with different registration poses are arranged, the registration relative pose of the registration poses respectively corresponding to the two registration devices in the registration apparatus are obtained, and the relative positioning pose of the first surgical cart relative to the second surgical cart is determined based on the first relative pose, the second relative pose, and the registration relative pose, thereby avoiding the case where the image collection devices cannot collect the FOV images containing the target object due to the limitations of the field of view of the image collection apparatus, and broadening the applicable scenarios of the embodiments of the present application.

The embodiments of a positioning apparatus for surgical cart of the present application are provided as follows. The positioning apparatus shares the same inventive concept as the positioning method for surgical cart in the above embodiments. For details not thoroughly described in the embodiments of the positioning apparatus for surgical cart, refer to the contents of the positioning method for surgical cart in the above embodiments.

FIG. 9 is a schematic view showing a structure of a positioning apparatus for surgical cart according to an embodiment of the present application. As shown in FIG. 9, the positioning apparatus includes a first relative pose determining module 510, a second relative pose determining module 520, and a relative positioning pose determining module 530.

The first relative pose determining module 510 is configured to obtain a first FOV image, correlated with a first surgical cart and a registration apparatus and collected by an image collection apparatus, and determine a first relative pose of the first surgical cart relative to the registration apparatus based on the first FOV image.

The second relative pose determining module 520 is configured to obtain a second FOV image, correlated with a second surgical cart and the registration apparatus and collected by the image collection apparatus, and determine a second relative pose of the registration apparatus relative to the second surgical cart based on the second FOV image.

The relative positioning pose determining module 530 is configured to determine a relative positioning pose of the first surgical cart relative to the second surgical cart based on the first relative pose and the second relative pose.

In the technical solutions of the embodiments, the first FOV image, correlated with the first surgical cart and the registration apparatus and collected by an image collection apparatus, is obtained, and the first relative pose of the first surgical cart relative to the registration apparatus is determined based on the first FOV image. The second FOV image, correlated with a second surgical cart and the registration apparatus and collected by the image collection apparatus, is obtained, and the second relative pose of the registration apparatus relative to the second surgical cart is determined based on the second FOV image, and the relative positioning pose of the first surgical cart relative to the second surgical cart is determined based on the first relative pose and the second relative pose, thereby avoiding the data interference between multiple sensor devices in positioning methods of the related art, and improving the positioning accuracy of the surgical carts while reducing the positioning complexity.

Exemplarily, the first relative pose determining module 510 is configured to: obtain object pixel data of a target object in the first FOV image, and determine the first relative pose of the first surgical cart relative to the registration device by using the efficient perspective-n-point camera pose estimation algorithm, based on the object pixel data, and a camera intrinsic matrix and a distortion matrix of the image collection device. The target object is the registration apparatus or the identification pattern disposed on the registration apparatus.

Exemplarily, a pose collecting module is arranged in the registration apparatus. Correspondingly, the relative positioning pose determining module 530 includes following units.

A first registration pose determining unit is configured to obtain a first registration pose corresponding to the first FOV image and a second registration pose corresponding to the second FOV image each collected by the pose collecting module.

A registration relative pose determining unit is configured to determine a registration relative pose based on the first registration pose and the second registration pose.

A first relative positioning pose determining unit is configured to determine the relative positioning pose of the first surgical cart relative to the second surgical cart based on the first relative pose, the second relative pose, and the registration relative pose.

Exemplarily, the first relative positioning pose determining unit is configured to:
obtain a change time interval corresponding to the first registration pose and the second registration pose, and a motion angular velocity and a motion acceleration;
obtain an angular displacement of the registration relative pose by performing integration on the motion angular velocity over the change time interval;
obtain a translational displacement of the registration relative pose by performing double integration on the motion acceleration over the change time interval.

Exemplarily, the registration apparatus includes two registration devices with different registration poses. The relative positioning pose determining module 530 includes a second relative positioning pose determining unit, which is configured to obtain a registration relative pose of registration poses respectively corresponding to two registration devices in the registration apparatus, and determine the relative positioning pose of the first surgical cart relative to the second surgical cart based on the first relative pose, the second relative pose, and the registration relative pose.

The positioning apparatus for surgical cart provided in the embodiments of the present application can perform the positioning method for surgical cart provided in any embodiment of the present application, and includes corresponding function modules for performing the method and achieves beneficial effects of the method.

Another embodiment of the present application provides a computer-readable storage medium. Computer instructions are stored on the computer-readable storage medium, and the computer instructions, when executed by a processor, cause the processor to implement the positioning method for surgical cart of any embodiment of the present application.

In the context of the present application, the computer-readable storage medium can be a tangible medium that can contain or store a computer program to be used by an instruction execution system, apparatus, or device; or to be used by combining the instruction execution system, apparatus, or device. The computer-readable storage medium may include, but is not limited to, electronic, magnetic, optical, electromagnetic, infrared, or semiconductor systems, apparatuses, or devices, or any suitable combination thereof. Exemplarily, the computer-readable storage medium may be a machine-readable signal medium. More specific examples of the machine-readable storage medium shall include an electrical connection based on one or more wires, a portable computer disk, a hard disk, a Random Access Memory (RAM), a Read-Only Memory (ROM), an Erasable Programmable Read-Only Memory (EPROM or Flash memory), an optical fiber, a portable Compact Disc Read-Only Memory (CD-ROM), an optical storage device, a magnetic storage device, or any suitable combination thereof. The computer-readable storage medium may be a non-transitory computer-readable storage medium.

For providing an interaction with a user, the systems and techniques described here can be implemented on an electronic device having: a display device (e.g., a Cathode Ray Tube (CRT) or a Liquid Crystal Display (LCD) monitor) for displaying information to the user; and a keyboard and a pointing device (e.g., a mouse or a trackball) by which the user can provide input to the electronic device. Other kinds of devices can also be used to provide the interaction with the user. For example, feedback provided to the user can be any form of sensory feedback (e.g., visual feedback, auditory feedback, or tactile feedback). Inputs from the user can be received in any form, including acoustic, speech, or tactile input.

The systems and techniques described herein can be implemented in a computing system (e.g., serving as a data server) that includes back-end components, a computing system (e.g., an application server) that includes middleware components, a computing system (e.g., a client computer having a graphical user interface or a Web browser, through which the user can interact with an implementation of the systems and the techniques described herein) that includes front-end components, or a computing system that includes any combination of such back-end, middleware, or front-end components. The components of the system can be interconnected by any form or medium of digital data communication (e.g., a communication network). An example of the communication network includes a Local Area Network (LAN), a Wide Area Network (WAN), a blockchain network, and the Internet.

The computing system can include a client and a server. The client and the server are generally remote from each other and typically interact through the communication network. The relationship of the client and the server generated by virtue of computer programs running on the respective computers and having a client-server relationship to each other. The server can be a cloud server, also known as a cloud computing server or a cloud host, and it is a host product in a cloud computing service system, which avoids the difficulties in management and weak business scalability existing in physical hosts and VPS services in the related art.

It should be understood that steps can be reordered, added, or deleted based on various forms of processes shown above. For example, the multiple steps described in the present application may be performed in parallel, sequentially, or in different orders, which is not limited herein, as long as the anticipated results of the technical solutions of the present application can be realized.

## Claims

1. A positioning method for surgical cart, comprising:
obtaining a first field-of-view (FOV) image, correlated with a first surgical cart and a registration apparatus and collected by an image collection apparatus, and determining a first relative pose of the first surgical cart relative to the registration apparatus based on the first FOV image;
obtaining a second FOV image, correlated with a second surgical cart and the registration apparatus and collected by the image collection apparatus, and determining a second relative pose of the registration apparatus relative to the second surgical cart based on the second FOV image; and
determining a relative positioning pose of the first surgical cart relative to the second surgical cart based on the first relative pose and the second relative pose.

2. The method according to claim 1, wherein determining the first relative pose of the first surgical cart relative to the registration apparatus based on the first FOV image comprises:
obtaining object pixel data of a target object in the first FOV image; and
determining the first relative pose of the first surgical cart relative to the registration apparatus by using an efficient perspective-n-point camera pose estimation algorithm, based on the object pixel data, and a camera intrinsic matrix and a distortion matrix of the image collection apparatus, wherein the target object is the registration apparatus or an identification pattern disposed on the registration apparatus.

3. The method according to claim 1, wherein a pose collecting module is arranged on the registration apparatus, and determining the relative positioning pose of the first surgical cart relative to the second surgical cart based on the first relative pose and the second relative pose comprises:
obtaining a first registration pose corresponding to the first FOV image and a second registration pose corresponding to the second FOV image, which are collected by the pose collecting module;
determining a registration relative pose based on the first registration pose and the second registration pose; and
determining the relative positioning pose of the first surgical cart relative to the second surgical cart based on the first relative pose, the second relative pose, and the registration relative pose.

4. The method according to claim 3, wherein determining the registration relative pose based on the first registration pose and the second registration pose comprises:
obtaining a change time interval corresponding to the first registration pose and the second registration pose, and a motion angular velocity and a motion acceleration;
obtaining an angular displacement of the registration relative pose by performing integration on the motion angular velocity over the change time interval; and
obtaining a translational displacement of the registration relative pose by performing double integration on the motion acceleration over the change time interval.

5. The method according to claim 1, wherein the registration apparatus comprises two registration devices with different registration poses, and determining the relative positioning pose of the first surgical cart relative to the second surgical cart based on the first relative pose and the second relative pose comprises:
obtaining a registration relative pose of registration poses respectively corresponding to the two registration devices in the registration apparatus; and
determining the relative positioning pose of the first surgical cart relative to the second surgical cart based on the first relative pose, the second relative pose, and the registration relative pose.

6. A positioning apparatus for surgical cart, comprising:
a first relative pose determining module, configured to obtain a first field-of-view (FOV) image correlated with a first surgical cart and a registration apparatus and collected by an image collection apparatus, and determine a first relative pose of the first surgical cart relative to the registration apparatus based on the first FOV image;
a second relative pose determining module, configured to obtain a second FOV image correlated with a second surgical cart and the registration apparatus and collected by the image collection apparatus, and determine a second relative pose of the registration apparatus relative to the second surgical cart based on the second FOV image; and
a relative positioning pose determining module, configured to determine a relative positioning pose of the first surgical cart relative to the second surgical cart based on the first relative pose and the second relative pose.

7. A positioning system for surgical cart, comprising a first surgical cart, a second surgical cart, an image collection apparatus, a registration apparatus, and a control device;
wherein the image collection apparatus is configured to collect a first FOV image correlated with the first surgical cart and the registration apparatus, and collect a second FOV image correlated with the second surgical cart and the registration apparatus; and
the control device comprises at least one processor, and a memory communicating with the at least one processor; wherein a computer program executable by the at least one processor is stored in the memory; and the computer program, when executed by the at least one processor, causes the at least one processor to execute the positioning method for surgical cart according to any one of claims 1 to 5.

8. The positioning system for surgical cart according to claim 7, wherein:
the registration apparatus comprises a registration device; and at least one identification pattern is disposed in the registration apparatus, or the registration apparatus has a display function for at least one identification pattern; and
the image collection apparatus comprises two image collection devices, and the two image collection devices are arranged on the first surgical cart and the second surgical cart respectively, and the first FOV image and the second FOV image each contain the identification pattern.

9. The positioning system for surgical cart according to claim 7, wherein the registration apparatus comprises a registration device, the number of image collection devices in the image collection apparatus is one, the image collection device is arranged in the registration apparatus, an identification pattern is disposed on each of the first surgical cart and the second surgical cart, the first FOV image is an FOV image containing the identification pattern on the first surgical cart, and the second FOV image is an FOV image containing the identification pattern on the second surgical cart.

10. The positioning system for surgical cart according to claim 7, wherein the registration apparatus comprises two registration devices with different registration poses, the number of image collection devices in the image collection apparatus is two; one image collection device or one identification pattern is disposed on each of the first surgical cart and the second surgical cart.

11. A computer-readable storage medium, wherein computer instructions are stored on the computer-readable storage medium, and the computer instructions, when executed by a processor, are configured to execute the positioning method for surgical cart according to any one of claims 1 to 5.
